(19) Europäisches Patentamt / European Patent Office / Office européen des brevets

(11) **EP 3 846 171 B1**

(12) **EUROPEAN PATENT SPECIFICATION**

(45) Date of publication and mention of the grant of the patent:
**20.05.2026 Bulletin 2026/21**

(21) Application number: **20217973.5**

(22) Date of filing: **31.12.2020**

(51) International Patent Classification (IPC):
**G16C 20/30** (2019.01)    **G16C 20/60** (2019.01)

(52) Cooperative Patent Classification (CPC):
**G06N 3/08; G06N 3/045; G06N 3/0499; G06N 3/09; G16C 20/50; G16C 20/64; G16C 20/70;** G06N 3/048; G16C 20/40

(54) **METHOD AND APPARATUS FOR NEW DRUG CANDIDATE DISCOVERY**

VERFAHREN UND VORRICHTUNG ZUR ERKENNUNG NEUER ARZNEIMITTELKANDIDATEN

PROCÉDÉ ET APPAREIL PERMETTANT DE DÉCOUVRIR DE NOUVEAUX MÉDICAMENTS CANDIDATS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.12.2019 KR 20190179469**
**17.12.2020 KR 20200177206**

(43) Date of publication of application:
**07.07.2021 Bulletin 2021/27**

(73) Proprietor: **Korea University Research and Business Foundation**
**Seoul 02841 (KR)**

(72) Inventors:
• **KANG, Jaewoo**
  **04765 Seoul (KR)**
• **JEON, Min Ji**
  **05555 Seoul (KR)**
• **CHANG, Bu Ru**
  **02827 Seoul (KR)**
• **PARK, Jung Soo**
  **05823 Seoul (KR)**
• **PARK, Sung Joon**
  **02855 Seoul (KR)**
• **KIM, Sun Kyu**
  **02843 Seoul (KR)**

(74) Representative: **Manitz Finsterwald**
**Patent- und Rechtsanwaltspartnerschaft mbB**
**Martin-Greif-Straße 1**
**80336 München (DE)**

(56) References cited:
**US-A1- 2019 114 390     US-A1- 2019 164 632**

• YONI DONNER ET AL: "Drug Repurposing Using Deep Embeddings of Gene Expression Profiles", MOLECULAR PHARMACEUTICS, vol. 15, no. 10, 12 July 2018 (2018-07-12), US, pages 4314 - 4325, XP055769831, ISSN: 1543-8384, DOI: 10.1021/acs.molpharmaceut.8b00284
• ALIPER ALEXANDER ET AL: "Deep Learning Applications for Predicting Pharmacological Properties of Drugs and Drug Repurposing Using Transcriptomic Data", vol. 13, no. 7, 5 July 2016 (2016-07-05), US, pages 2524 - 2530, XP055800073, ISSN: 1543-8384, Retrieved from the Internet <URL:https://pubs.acs.org/doi/pdf/10.1021/acs.molpharmaceut.6b00248> DOI: 10.1021/acs.molpharmaceut.6b00248

**Description**

**BACKGROUND**

1. Technical Field

The present disclosure relates to a new drug candidate material output apparatus and a method for outputting new drug candidate material for deriving a new drug candidate material by using a transcriptome phenotype.

2. Related Art

A new drug is developed by a process configured of a new drug discovery step and a new drug development step. The new drug discovery step includes a target identification, a candidate material design, an efficacy measurement, and a drug candidate material selection. The new drug development step includes a safety evaluation and a clinical trial of the drug material candidate. Although it takes a considerable amount of time and cost to commercialize a drug through the new drug discovery step and the new drug development step, it is known that a success rate thereof is not high.

In a new drug development pipeline, it is very important to identify a target protein suitable for a disease and find a molecule that binds to the target. Once the target for the disease is identified, a compound capable of binding to the target is discovered through high-efficiency screening, and a structural analog of the drug that binds to the target is also selected as a drug candidate material.

In this way, about 5,000 to 10,000 or more drugs are selected as drug candidate materials, but a success rate before being sold through experiments and verifications is less than 0.02%, so that a development cost and a development time of a new drug are increased.

As described above, the process of developing a new drug not only requires a lot of time and cost, but also it is a difficult process, and there is no guarantee that the new drug to be developed actually succeeds. In addition, research and development costs in a pharmaceutical industry are increasing, and productivity, which is calculated as a ratio of research and development costs to the number of newly approved drugs, has been steadily decreasing every year since the 1950s. Since the success of a new drug development depends on the selection of a new drug candidate, it is important to select a new drug candidate having a high probability of success in order to increase the productivity of the new drug development.

A traditional method used to discover a new drug candidate material in the new drug discovery step is a method for a discovery of the new drug candidate material based on a target. It proceeds with a discovery of the target protein which is a process of discovering major factors related to the disease, an effective material hit discovery which is a process of finding a compound that may physically bind to the target protein to inhibit a function thereof, and a lead optimization process of structurally optimizing the previously found effective material. Among the drug candidates discovered from the target protein, a drug that has an effect on cells, tissues, and individuals is finally selected through the development step.

However, the target-centered new drug development process is limited in that (1) the target protein hypothesis is essential, (2) even if the target protein hypothesis is found, drug development may be difficult if a compound has an undruggable target, and (3) even with a structure in which the compound may bind to the target protein, it is difficult to experimentally verify a myriad of compounds, and it takes a considerable amount of time of about 5.5 years or more to derive candidate materials. Specific examples are as follows.

For example, in the existing target-centered drug development method, a target protein is first set and a compound that binds to the protein is searched. However, if the target protein cannot be identified because the disease is not well understood, the process of developing a new drug that may treat the disease cannot be started. For example, in a case of Alzheimer's disease, it is difficult to search for new drug candidate materials because a clear target protein that acts as a factor of the disease cannot be identified.

In addition, the drug development may be difficult even in a case where the target protein that plays an important role in the treatment of the disease is known but a material that actually binds to the protein cannot be made. KRAS, which is widely known as a target protein for lung or colon cancer, has no binding site to which drugs may bind, so that a KRAS inhibitor does not currently exist. This means that no matter how high the understanding of the disease is, it is impossible to develop a new drug for this disease with the existing target-centered method.

As a technology related to the present disclosure is Korean Patent Laid-Open Publication No. 10-2018-0058648 (title of the invention: new drug candidate material discovery method and apparatus for targeting disorder-to-order transition site).

In this regard, a deep learning model which measures functional similarities between compounds based on gene expression data for each compound is disclosed in US 2019/0114390 A1 (Title: Drug repurposing based on deep embeddings of gene expression profiles) and YONI DONNER ET AL "Drug repurposing using deep embeddings of gene expression profiles", Molecular Pharmaceutics (2018), Vol. 15, No. 10, p. 4314-4325. And a system of predicting drug indications and drug response using an artificial intelligence (AI) deep learning model based on convergence of different types of information is disclosed in US 2019/0164632 A1 (Title: Drug indication and response prediction systems and method using ai deep learning based on convergence of different category data). And a method for classifying various drugs as therapeutics by a deep neural network (DNN) based on transcriptional profile information is disclosed in ALIPER ALEXANDER ET AL" Deep Learning Applications for Predicting Pharmacological Properties of Drugs and Drug Repurposing Using Transcriptomic Data", Molecular Pharmaceutics (2016), Vol. 13, No. 7, p. 2524-2530". These documents do not disclose a machine learning model in which an embedding vector for a chemical structure of a chemical compound and an embedding vector for an amount of a transcriptome change induced by each chemical compound are located in a same vector space.

## SUMMARY

In order to solve the above-described problems, an object of an example of the present disclosure is to provide a new drug candidate material output apparatus and a method for outputting new drug candidate material capable of outputting a new drug candidate material through a drug learning model learned by inputting data of a change in an amount of a transcriptome and data of each chemical compound.

However, technical problems to be solved by the present example are not limited to the technical problems as described above, and other technical problems may exist.

As technical means for solving the above technical problems, according to an example of the present disclosure, there is provided an apparatus for outputting one or more new drug candidates, including: a communication module; a memory in which a program for outputting one or more new drug candidates is stored; and a processor executing the program. The program provides a machine

learning model in which an embedding vector for a chemical structure of a chemical compound and an embedding vector for an amount of a transcriptome change induced by each chemical compound are located in a same vector space, outputs an amount of the transcriptome change that matches the embedding vector for the chemical structure of the new material input to the machine learning model, or outputs one or more drugs that match the embedding vector of the transcriptome change that is input to the machine learning model, wherein when data on an amount of the transcriptome before administration of the chemical compound is input, the machine learning model is iteratively learned to minimize a difference between data of the amount of the transcriptome change output by the machine learning model and data of an amount of the transcriptome after administration of an actual chemical compound, and wherein the amount of the transcriptome change represents the difference between the amount of the transcriptome before administration of the chemical compound and the amount of the transcriptome after administration of the chemical compound.

In addition, according to another embodiment of the present disclosure, there is provided a computer-implemented method for constructing a machine learning model of a computing device for discovering one or more new drug candidates, the method

including: a step of constructing a machine learning model in which an embedding vector for a chemical structure of a chemical compound and an embedding vector for an amount of a transcriptome change induced by each chemical compound are located in a same vector space; and a step of executing iterative learning to minimize a difference between data of an amount of the transcriptome change inferred by the machine learning model and data of an amount of the transcriptome after the administration of an actual chemical compound when data on an amount of the transcriptome before the administration of the chemical compound is input to the machine learning model, and wherein the amount of the transcriptome change represents the difference between the amount of the transcriptome before administration of the chemical compound and the amount of the transcriptome after administration of the chemical compound.

In addition, according to further another embodiment of the present disclosure, there is provided a computer-implemented method for outputting one or more new drug candidates through an inference process of a machine learning model of a computing device, the method including: a step of providing a machine learning model in which an embedding vector for a chemical structure of a chemical compound and an embedding vector for an amount of a transcriptome change induced by each chemical compound are located in a same vector space; a step of inputting an embedding vector for a chemical structure of a new material or an embedding vector for an amount of a transcriptome change to the machine learning model; and a step of outputting, by the machine learning model, an amount of the transcriptome change that matches the embedding vector for the chemical structure of the new material, or outputting one or more drugs that match the embedding vector for the amount of the transcriptome change, wherein, when data on an amount of the transcriptome before administration of the chemical compound is input, the machine learning model is iteratively learned to minimize a difference between data of the amount of the transcriptome change output by the machine learning model and data of an amount of the transcriptome after administration of an actual chemical compound, and wherein the amount of the transcriptome change represents the difference between the amount of the transcriptome before administration of the chemical compound and the amount of the transcriptome after administration of the chemical compound.

According to the above-described means for solving the problems of the present disclosure, it is possible to greatly reduce a time and cost consumed in the discovery step of discovering a new drug candidate.

In addition, new drugs may be developed even in a case where a target protein hypothesis does not exist or the target protein is known but a material that actually binds to the protein cannot be made.

## BRIEF DESCRIPTION OF THE DRAWINGS

FIG. 1 is a diagram illustrating a configuration of a new drug candidate material output apparatus according to an example of the present disclosure.

FIG. 2 is an exemplary diagram for explaining an operation of the new drug candidate material output apparatus according to an example of the present disclosure.

FIG. 3 is a flowchart for explaining an operation of a method for outputting a new drug candidate material according to an example of the present disclosure.

## DETAILED DESCRIPTION OF THE EMBODIMENTS

Hereinafter, examples of the present application will be described in detail with reference to the accompanying drawings so that those of ordinary skill in the art may easily implement the present application. However, the present application may be implemented in various different forms and is not limited to the examples described herein. In the drawings, portions not related to the description are omitted in order to clearly describe the present application, and similar reference numerals are attached to similar portions throughout the specification.

Throughout this specification, when a portion is said to be "connected" with another portion, this includes not only a case that it is "directly connected", but also a case where it is "electrically connected" with another element interposed therebetween.

Throughout this specification, when a member is positioned "on" another member, this includes not only a case where a member is in contact with another member, but also a case where further another member exists between two members.

Hereinafter, an example of the present disclosure will be described in detail with reference to the accompanying drawings.

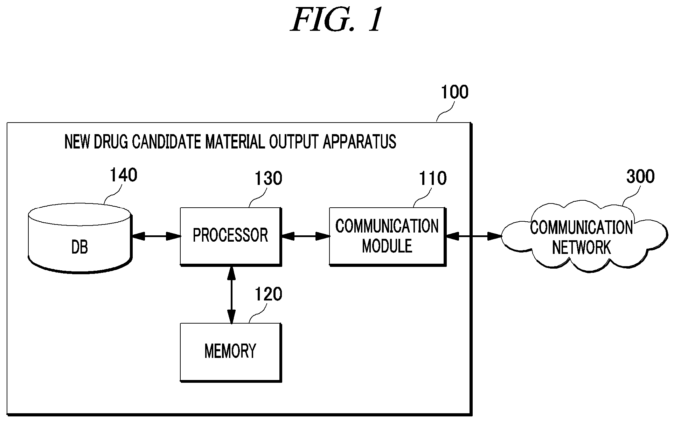

FIG. 1 is a diagram illustrating a configuration of a new drug candidate material output apparatus according to an example of the present disclosure.

As illustrated in the drawing, a new drug candidate material output apparatus 100 may include a communication module 110, a memory 120, a processor 130, and a database 140. The new drug candidate material output apparatus 100 is basically configured of a computing device, and further includes a power supply unit, various input devices and output devices, and the like which are not illustrated.

The communication module 110 may transmit and receive data on a chemical structure of a chemical compound with an external computing device, or data on change information on an amount of a transcriptome induced by a chemical compound as matching therewith. The communication module 110 may be a device including hardware and software necessary to transmit and receive a signal such as a control signal or a data signal through wired/wireless connection with another network device.

A new drug candidate material output program is stored in the memory 120. The new drug candidate output program provides a drug learning model in which an embedding vector for the chemical structure of the chemical compound and an embedding vector for the change information on the amount of the transcriptome induced by each chemical compound are located in a same vector space, and outputs a new drug candidate material based on a query input through the input device, the communication module 110, or the like. In this case, the input query may be data on a chemical structure of a new material or the change information on the amount of the transcriptome to be a target.

In addition, in the new drug candidate material output program, a logic for constructing a drug learning model, a learning model update process for executing a new learning process on the constructed drug learning model, or the like may be additionally performed.

The memory 120 stores various types of data generated during the execution of an operating system for driving the new drug candidate material output apparatus 100 or a missing data prediction program.

In this case, the memory 120 collectively refers to a nonvolatile storage device that continuously maintains stored information even if power is not supplied and a volatile storage device that requires power to maintain the stored information.

In addition, the memory 120 may execute a function of temporarily or permanently storing data processed by the processor 130. Here, the memory 120 may include magnetic storage media or flash storage media in addition to the volatile storage device that requires power to maintain the stored information, but the scope of the present disclosure is not limited thereto.

The processor 130 executes a program stored in the memory 120 and controls an entire process according to the execution of the new drug candidate material output program. Each operation performed by the processor 130 will be described later in more detail.

The processor 130 may include all types of devices capable of processing data. For example, it may refer to a data processing device built in hardware having a circuit physically structured to execute a function represented by codes or instructions included in a program. As an example of the data processing device built in hardware as described above, processing devices such as a microprocessor, a central processing unit (CPU), a processor core, a multiprocessor, an application-specific integrated circuit (ASIC), and a field programmable gate array (FPGA) may be covered, but the scope of the present disclosure is not limited thereto.

The database 140 stores or provides data necessary for the new drug candidate material output apparatus under a control of the processor 130. The database 140 may be included as a configuration element separated from the memory 120 or may be constructed in a partial region of the memory 120.

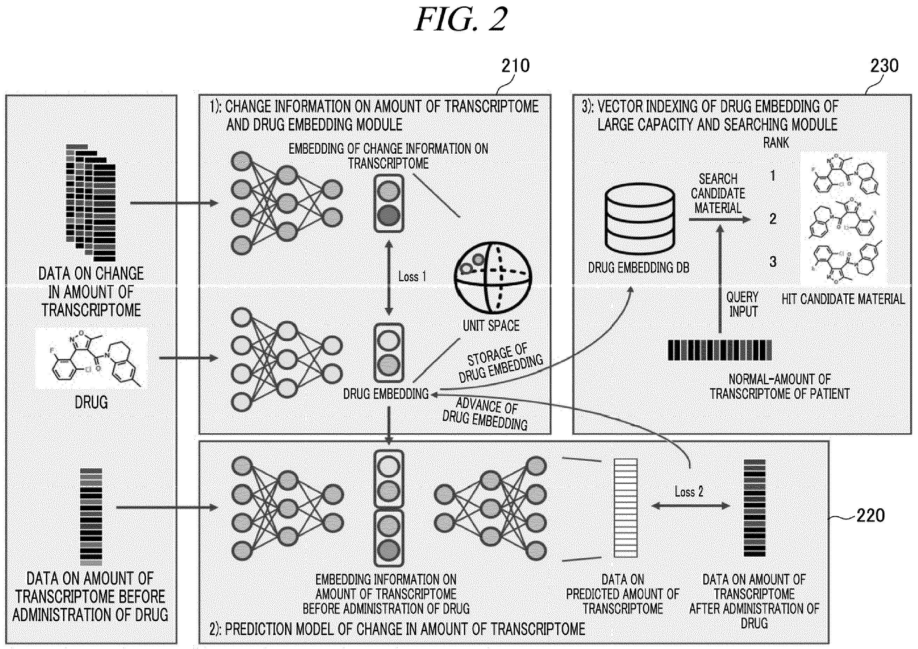

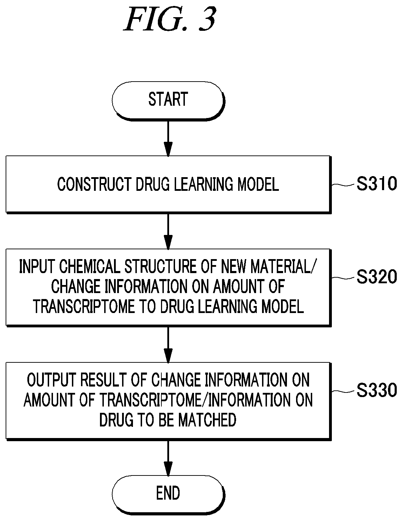

FIG. 2 is an exemplary diagram for explaining an operation of the new drug candidate material output apparatus according to an example of the present disclosure and FIG. 3 is a flowchart for explaining an operation of a method for outputting a new drug candidate material according to an example of the present disclosure.

First, a method for constructing a drug learning model of the new drug candidate material output apparatus 100 will be described (S310).

The drug learning model of the new drug candidate material output apparatus 100 may be configured of a multi-layered artificial neural network for learning the change information of the amount of the transcriptome and a multi-layered artificial neural network for learning chemical structure information on the chemical compound.

Each layer may be configured of a basic perceptron layer (fully-connected layer), and use a graph neural network (GNN) to generate an embedding vector centering on the chemical structure of the chemical compound. As an example, the multi-layered artificial neural network for learning the change information of the amount of the transcriptome and the multi-layered artificial neural network for learning the chemical structure information on the chemical compound each have 4 layers, and may be implemented in a form of neural networks fully connected in which embedding vectors of the same dimension are generated as an output.

The drug learning model of the new drug candidate material output apparatus 100 disposes, in the same vector space, the embedding vector for the chemical structure of the chemical compound and the embedding vector for the change information on the amount of the transcriptome induced by each chemical compound.

The change information on the amount of the transcriptome means a change in an expression level (amount of the transcriptome) of a plurality of genes in cells before and after the administration of the drug. The change in the amount of the transcriptome before and after the administration of the drug includes information on an effect of the drug on cells, that is, a complex interaction between the drug and all proteins in the cell, and between proteins and proteins. At this time, a degree of the change in the amount of the transcriptome may be defined as a distance from an average value of a Gaussian distribution configured of the amount of the transcriptome (gene-expression) of each gene before the administration of the drug to the amount of the transcriptome of each gene after the administration of the drug. That is, the degree of the change increases in proportion to the distance value. At this time, the distance between the two amounts of the transcriptomes may be obtained through the Gaussian as follows.

$$K(x_g, m_g) = \exp\left(-\frac{\|x_g - m_g\|^2}{2\sigma_g^2}\right)$$

Where, $m_g$ refers to the average of values of the amounts of the transcriptomes of a gene g before the administration of the drug, $\sigma_g$ refers to a sample average of the values of the amounts of the transcriptomes of the gene g before the administration of the drug, and $x_g$ refers to the value of the amount of the transcriptome of the gene g after the administration of the drug.

For example, learning data that contains the change information on the amount of the transcriptome when the drug is administered to a cancer cell line may be considered, which is assumed to include 978 representative genes. At this time, the change information on the amount of the transcriptome according to specific the administration of the drug includes a distance value from the average value of the Gaussian distribution for the transcriptome of each gene for each of total 978 genes to the amount of the transcriptome of each gene after the administration of the drug.

Using this, it is possible to solve the problem that new drug development was impossible by the existing target-oriented new drug development method. For example, in a case where there is no disease-related target protein hypothesis, drug candidate materials that may induce a gene expression pattern of a patient group to a gene expression pattern of a normal group may be discovered. In addition, even if the disease-related target protein hypothesis is known, in a case where drug development is difficult because the target protein is unable to bind, a candidate material capable of inducing a change in gene expression due to target gene knockdown may be discovered.

As illustrated in the drawing, the embedding vector for the chemical structure of the chemical compound and the embedding vector for the change information on the amount of the transcriptome generating when the chemical compound is injected as a drug are disposed in the same vector space (for example, a surface of a unit hypersphere), so that the drug learning model 210 is constructed.

At this time, since the change information on the amount of the transcriptome before and after the administration of the drug includes all information on the reaction of the drug including an off-target effect, the change information on the amount of the transcriptome and the drug inducing the change are embedded to be located in the same vector space, so that it is possible to capture abstract characteristics of the drug effect. In addition, since it is an embedding module based on drug effects, even a drug having a new chemical structure may be discovered as a new drug candidate based on drug properties. In addition, since the embedding module of the present disclosure uses the chemical structure of the drug as an input, not only existing chemical compounds with known structures but also all synthesizable compounds to be known in the future may be expressed as embedding based on drug effects without an incurring separate process and cost.

In addition, the drug learning model of the present disclosure constructs, in the same vector space, the embedding vector for the chemical structure of the chemical compound and the embedding vector for the change information on the amount of the transcriptome that occurs when the chemical compound is injected as a drug, and uses a triplet loss function as a loss function therefor. In the loss function, an embedding vector $f^a$ representing the change information on the amount of the transcriptome generating according to the administration of a specific chemical compound, an embedding vector $f^p$ representing the chemical compound that induces the change in the amount of the transcriptome, and an embedding vector $f^n$ representing a chemical compound that does not induce the change in the amount of the transcriptome are used.

Triplet_loss(Anchor, Positive, Negative)

$$Loss = \sum_{i=1}^{N} \max\left(Dist\left(f_i^a, f_i^p\right) - Dist\left(f_i^a, f_i^n\right) + \alpha, 0\right)$$

That is, the triplet loss function is calculated based on a value obtained by subtracting a distance between the embedding vector $f^a$ representing the change information on the amount of the transcriptome and the embedding vector $f^n$ representing the chemical compound that does not induce the change in the amount of the transcriptome from a distance between the embedding vector $f^a$ representing the change information on the amount of the transcriptome and the embedding vector $f^p$ representing the chemical compound that induces the change in the amount of the transcriptome.

In this case, a represents a margin value set by a model designer, i represents the number of learning times or the identification number of a sample, and N represents the number of pairs of each chemical compound that induces each change in the amount of the transcriptome and change information on the amount of the transcriptome. For example, if the learning data includes 310,114 change information on the amount of the transcriptome tested for a total of 21,220 drugs and 82 cell lines, N may be 310,114 corresponding to the total number of change information on the amount of the transcriptome.

A distance Dist(A,B) for any two embedding vectors A and B uses a negative cosine similarity, and is defined as follows.

$$Dist(A, B) = 1 - \frac{A \cdot B}{\|A\| \times \|B\|}$$

The triplet loss function minimizes the distance between the embedding vector representing the change information on the amount of the transcriptome and the embedding vector for the chemical compound that induces the change in the amount of the transcriptome, and the distance with the embedding vector for the chemical compound that does not induce the change in the amount of the transcriptome is configured to maximize.

In order to further maximize the performance of the loss function of the present disclosure, a double triplet loss function may be used as follows.

$$Loss = \sum_{i=1}^{N} \max\left(Dist\left(f_i^a, f_i^p\right) - Dist\left(f_i^a, f_i^n\right) + \alpha, 0\right)$$

$$+ \max\left(Dist\left(f_i^p, f_i^a\right) - Dist\left(f_i^p, f_i^n\right) + \alpha, 0\right)$$

In contrast to the triplet loss function described above, a term is added, which is obtained by subtracting the distance between the embedding vector $f^p$ representing the chemical compound that induces the change in the amount of the transcriptome and the embedding vector $f^n$ representing the chemical compound that does not induce the change in the amount of the transcriptome from the distance between the embedding vector $f^p$ representing the chemical compound that induces the change in the amount of the transcriptome in rear and the embedding vector $f^a$ representing the change information on the amount of the transcriptome.

If the triplet loss function described above is used, in a case where the distance between the embedding vector $f^a$ representing the change information on the amount of the transcriptome and the embedding vector $f^n$ representing the chemical compound that does not induce the change in the amount of the transcriptome is very long, the loss function value is counted as zero. In this case, there may be a case where the distance between the embedding vector $f^a$ representing the change information on the amount of the transcriptome and the embedding vector $f^p$ representing the chemical compound that induces the change in the amount of the transcriptome is not narrowed.

If the double triplet loss function is used, even if the term in front becomes 0, it is possible to narrow the distance between the embedding vector $f^p$ representing the chemical compound that induces the change in the amount of the transcriptome and the embedding vector $f^a$ representing the change information on the amount of the transcriptome through the term added to the rear.

On the other hand, in order to further advance the learning model constructed as described above, an iterative learning step may be additionally performed (220).

That is, when data on the amount of the transcriptome before the administration of the chemical compound is input to the drug learning model, iterative learning is executed to minimize a difference between the data of the amount of the transcriptome inferred by the drug learning model and the data of the amount of the transcriptome after the administration of the actual chemical compound. To this end, an operation of updating the weight of the learning model may be performed by using a predetermined loss function so that the difference between the data on the amount of the transcriptome output from the learning model and the data on the amount of the transcriptome after the administration of the drug is minimized.

Next, the embedding vector for the chemical structure of the new material or the change information on the amount of the transcriptome to be the target is input as a query for the thus constructed learning model (S320).

In the present disclosure, embedding vectors are obtained for all known compounds, and indexes are prepared in advance so that they may be quickly searched and stored in a DB or the like. Existing known compounds are about 1.3 billion based on those registered in a Zinc15 DB. Including these compounds, it is implemented to allow additional indexing of compounds to be added in the future. If a similar vector search system constructed in this way is used, it is possible to find a compound most similar to the query vector among various compounds expressed as dense vectors. In this case, the query vector may be an embedding vector for a chemical structure of a new material, or be an embedding vector for the change information on the amount of the transcriptome after the administration of the drug.

For example, in order to develop a new medical use of a drug under development or on sale, such as drug repositioning, the embedding vector of the drug may be used as a query vector, the change information on the amount of the transcriptome may be used as the query in order to select a new drug candidate executing a required function such as drug screening. In this case, it is possible to select a drug candidate group within a short period of time, in units of several seconds, as for a similarity search time for about 1.3 billion compounds.

Next, as an output for the query vector, the drug learning model outputs the result of change information on the amount of the transcriptome that matches the embedding vector for the chemical structure of the new material, or outputs information on one or more drugs matching the change information on the amount of the transcriptome to be the target (S330).

As illustrated in FIG. 2, when the change information on the amount of the transcriptome is input as the query, the drug learning model outputs a drug candidate material matching therewith. In addition, when the embedding vector for the chemical structure of a new material is input, the drug learning model outputs a result of matching change information on the amount of the transcriptome.

On the other hand, in the present disclosure, as described above, a drug candidate material matching therewith may be output based on the change information on the amount of the transcriptome. In particular, in a case where the target protein is determined, the change information on the amount of the transcriptome may be specified based on a difference between the amount of the transcriptome (reference amount of the transcriptome) before knock out (KO)/ knock down (KD) of the gene of the target protein and the amount (amount of induction transcriptome) of the transcriptome after KO/KD of the gene of the target protein. Then, by inputting the thus obtained change information on the amount of the transcriptome to the drug learning model, a drug candidate material may be output.

Alternatively, in a case where information on the amount of the transcriptome of the normal group and the target disease group is given, the change information on the amount of the transcriptome may be specified based on a difference between the amount (reference amount of the transcriptome) of the transcriptome of the disease group and the amount (amount of the induction transcriptome) of the transcriptome of the normal group. Then, by inputting the thus obtained change information on the amount of the transcriptome to the drug learning model, a drug candidate material may be output.

On the other hand, a variance of an absolute value of the change amount of the amount of the transcriptome may be significantly different from a variance of the value of the change amount of the amount of the transcriptome used during model learning, depending on an observation method for the value. In a case where the value is used as an input of the drug learning model, it may be difficult to obtain expected results because the value is significantly different from that in a model learning environment. To solve this problem, it is necessary to generally match the deviation of the change information on the amount of the transcriptome input as the query with the deviation of the learning data of the change information on the amount of the transcriptome used in the learning process of the drug learning model.

To this end, each gene is listed in order of the size of a T-statistic through a T-test for the amount of the reference transcriptome and the amount of the induction transcriptome constituting the newly input change information of the amount of transcriptome. This is the same as listing genes in order of the size of the change in the amount of the transcriptomes for the newly input transcriptome information. Next, with respect to the genes listed in order of the size of the amount of the change in the transcriptome, the values of the amounts of the changes in the transcriptome of the learning data are mapped in order of the size. For example, for a gene in which the amount of the transcriptome is most decreased in the amounts of the induction transcriptomes compared to the amount of the reference transcriptome, a negative absolute value gives the largest value among the changes in the transcriptomes of the learning data. Through this method, for the information on the newly introduced transcriptome, it is possible to generate input data having the same level of deviation as the deviation of the value of the learning data while maintaining the order of genes based on the amount of the change in the transcriptome.

In the drug learning model, when an embedding vector of adjusted change information on the amount of the transcriptome is input, compounds having the closest vector values are searched from the previously constructed compound embedding database. In this case, a general distance function such as a Euclidean distance or a cosine similarity may be used as the distance between vectors. Based on this, compounds having the closest vector values are derived as drug candidates that induce an expected effect of the change in the amount of the transcriptome.

An example of the present disclosure may also be implemented in a form of a recording medium including instructions executable by a computer, such as a program module executed by a computer. The computer-readable medium may be any available medium that may be accessed by a computer, and includes both volatile and nonvolatile media, and removable and non-removable media. Further, the computer-readable medium may include a computer storage medium. The computer storage medium includes both volatile and nonvolatile, removable and non-removable media implemented in any method or technology for storage of information such as computer readable instructions, data structures, program modules, or other data.

Although the methods and systems of the present disclosure are described in connection with specific examples, some or all of their configuration elements or operations may be implemented by using a computer system having a general-purpose hardware architecture.

The foregoing description of the present application is for illustrative purposes only.

The scope of the present application is indicated by the claims.

**Description of Symbols**

100: new drug candidate material output apparatus
110: communication module
120: memory
130: processor
140: database

# EP 3 846 171 B1

## Claims

1. An apparatus for outputting one or more new drug candidates, comprising:

   a communication module;
   a memory in which a program for outputting one or more new drug candidates is stored; and
   a processor executing the program,
   wherein the program provides a machine learning model in which an embedding vector for a chemical structure of a chemical compound and an embedding vector for an amount of a transcriptome change induced by each chemical compound are located in a same vector space, outputs an amount of the transcriptome change that matches the embedding vector for the chemical structure of the new material input to the machine learning model, or outputs one or more drugs that match the embedding vector for the amount of the transcriptome change that is input to the machine learning model,
   wherein when data on an amount of the transcriptome before administration of the chemical compound is input, the machine learning model is iteratively learned to minimize a difference between data of the amount of the transcriptome change output by the machine learning model and data of an amount of the transcriptome after administration of an actual chemical compound, and
   wherein the amount of the transcriptome change represents the difference between the amount of the transcriptome before administration of the chemical compound and the amount of the transcriptome after administration of the chemical compound.

2. The apparatus of claim 1,

   wherein the machine learning model is configured to minimize a distance between a first embedding vector representing the amount of the transcriptome change and an embedding vector of a chemical compound that induces the change in the amount of the transcriptome through a triplet loss function,
   and maximize a distance between the first embedding vector and an embedding vector of a chemical compound that does not induce a change in the amount of the transcriptome.

3. A computer-implemented method for constructing a machine learning model of a computing device for discovering one or more new drug candidates, the method comprising:

   a step of constructing a machine learning model in which an embedding vector for a chemical structure of a chemical compound and an embedding vector for an amount of a transcriptome change induced by each chemical compound are located in a same vector space; and
   a step of executing iterative learning to minimize a difference between data of an amount of the transcriptome change inferred by the machine learning model and data of an amount of the transcriptome after administration of an actual chemical compound when data on an amount of the transcriptome before administration of the chemical compound is input to the machine learning model, and
   wherein the amount of the transcriptome change represents the difference between the amount of the transcriptome before administration of the chemical compound and the amount of the transcriptome after administration of the chemical compound.

4. The computer-implemented method of claim 3,
   wherein in the step of constructing the machine learning model, a distance between a first embedding vector representing the amount of the transcriptome change and a embedding vector of a chemical compound that induces the change in the amount of the transcriptome through a triplet loss function is minimized, and a distance between the first embedding vector and an embedding vector of a chemical compound that does not induce a change in the amount of the transcriptome is maximized.

5. A computer-implemented method for outputting one or more drug candidates through an inference process of a machine learning model of a computing device, the method comprising:

   a step of providing a machine learning model in which an embedding vector for a chemical structure of a chemical compound and an embedding vector for an amount of a transcriptome change induced by each chemical compound are located in a same vector space;
   a step of inputting an embedding vector for a chemical structure of a new material or an embedding vector for an amount of a transcriptome change to the machine learning model; and

9

a step of outputting, by the machine learning model, an amount of a transcriptome change that matches the embedding vector for the chemical structure of the new material, or outputting one or more drugs that match the embedding vector for the amount of the transcriptome change,

wherein, when data on an amount of the transcriptome before administration of the chemical compound is input, the machine learning model is iteratively learned to minimize a difference between data of the amount of the transcriptome change output by the machine learning model and data of an amount of the transcriptome after administration of an actual chemical compound, and

wherein the amount of the transcriptome change represents the difference between the amount of the transcriptome before administration of the chemical compound and the amount of the transcriptome after administration of the chemical compound.

6. The computer-implemented method of claim 5,
wherein the machine learning model minimizes a distance between a first embedding vector representing the amount of the transcriptome change and an embedding vector of a chemical compound that induces the change in the amount of the transcriptome through a triplet loss function, and maximizes a distance between the first embedding vector and the embedding vector of the chemical compound that does not induce the change in the amount of the transcriptome.

7. A non-transitory computer-readable medium in which a program for executing the method of any one of claims 3 to 6 is recorded.

**Patentansprüche**

1. Vorrichtung zum Ausgeben eines oder mehrerer Arzneimittelkandidaten, umfassend:

ein Kommunikationsmodul;
einen Speicher, in dem ein Programm zum Ausgeben eines oder mehrerer Arzneimittelkandidaten gespeichert ist; und
einen Prozessor, der das Programm ausführt,
wobei das Programm ein Maschinenlernmodell bereitstellt, in welchem ein Einbettungsvektor für eine chemische Struktur einer chemischen Verbindung und ein Einbettungsvektor für ein Ausmaß einer durch jede chemische Verbindung induzierten Transkriptomänderung sich in einem gleichen Vektorraum befinden, ein Ausmaß der Transkriptomänderung ausgibt, das mit dem Einbettungsvektor für die in das Maschinenlernmodell eingegebene chemische Struktur des neuen Materials übereinstimmt, oder
ein oder mehrere Arzneimittel ausgibt, die mit dem Einbettungsvektor für das Ausmaß der Transkriptomänderung übereinstimmen, welches in das Maschinenlernmodell eingegeben wird,
wobei, wenn Daten zu einer Menge des Transkriptoms vor Verabreichung der chemischen Verbindung eingegeben werden, das Maschinenlernmodell iterativ trainiert wird, um eine Differenz zwischen Daten des von dem Maschinenlernmodell ausgegebenen Ausmaßes der Transkriptomänderung und Daten einer Menge des Transkriptoms nach Verabreichung einer tatsächlichen chemischen Verbindung zu minimieren, und
wobei das Ausmaß der Transkriptomänderung die Differenz zwischen der Menge des Transkriptoms vor Verabreichung der chemischen Verbindung und der Menge des Transkriptoms nach Verabreichung der chemischen Verbindung darstellt.

2. Vorrichtung nach Anspruch 1,
wobei das Maschinenlernmodell dazu ausgebildet ist, einen Abstand zwischen einem das Ausmaß der Transkriptomänderung darstellenden ersten Einbettungsvektor und einem Einbettungsvektor einer chemischen Verbindung, welche die Änderung der Menge des Transkriptoms induziert, durch eine Triplett-Verlustfunktion zu minimieren, und einen Abstand zwischen dem ersten Einbettungsvektor und einem Einbettungsvektor einer chemischen Verbindung, die keine Änderung der Menge des Transkriptoms induziert, zu maximieren.

3. Computerimplementiertes Verfahren zum Aufbauen eines Maschinenlernmodells einer Rechenvorrichtung zum Auffinden eines oder mehrerer neuer Arzneimittelkandidaten, wobei das Verfahren umfasst:

einen Schritt des Aufbauens eines Maschinenlernmodells, in welchem ein Einbettungsvektor für eine chemische Struktur einer chemischen Verbindung und ein Einbettungsvektor für ein durch jede chemische Verbindung induziertes Ausmaß einer Transkriptomänderung sich in einem gleichen Vektorraum befinden; und
einen Schritt des Durchführens von iterativem Lernen, um eine Differenz zwischen Daten eines durch das Maschinenlernmodell hergeleiteten Ausmaßes der Transkriptomänderung und Daten einer Menge des Transkriptoms nach Verabreichung einer tatsächlichen chemischen Verbindung zu minimieren, wenn Daten zu einer Menge des Transkriptoms vor Verabreichung der chemischen Verbindung in das Maschinenlernmodell eingegeben werden, und

wobei das Ausmaß der Transkriptomänderung die Differenz zwischen der Menge des Transkriptoms vor Verabreichung der chemischen Verbindung und der Menge des Transkriptoms nach Verabreichung der chemischen Verbindung darstellt.

**4.** Computer-implementiertes Verfahren nach Anspruch 3,
wobei in dem Schritt des Aufbauens des Maschinenlernmodells ein Abstand zwischen einem das Ausmaß der Transkriptomänderung darstellenden ersten Einbettungsvektor und einem Einbettungsvektor einer chemischen Verbindung, welche die Änderung der Menge des Transkriptoms induziert, durch eine Triplett-Verlustfunktion minimiert wird, und ein Abstand zwischen dem ersten Einbettungsvektor und einem Einbettungsvektor einer chemischen Verbindung, die keine Änderung der Menge des Transkriptoms induziert, maximiert wird.

**5.** Computer-implementiertes Verfahren zum Ausgeben eines oder mehrerer Arzneimittelkandidaten durch einen Herleitungsprozess eines Maschinenlernmodells einer Rechenvorrichtung, wobei das Verfahren umfasst:

einen Schritt des Bereitstellens eines Maschinenlernmodells, in welchem ein Einbettungsvektor für eine chemische Struktur einer chemischen Verbindung und ein Einbettungsvektor für ein Ausmaß einer durch jede chemische Verbindung induzierten Transkriptomänderung sich in einem gleichen Vektorraum befinden;
einen Schritt des Eingebens eines Einbettungsvektors für eine chemische Struktur eines neuen Materials oder eines Einbettungsvektors für ein Ausmaß einer Transkriptomänderung in das Maschinenlernmodell; und
einen Schritt des Ausgebens, durch das Maschinenlernmodell, eines Ausmaßes einer Transkriptomänderung, das mit dem Einbettungsvektor für die chemische Struktur des neuen Materials übereinstimmt, oder des Ausgebens eines oder mehrerer Arzneimittel, die mit dem Einbettungsvektor für das Ausmaß der Transkriptomänderung übereinstimmen,
wobei, wenn Daten zu einer Menge des Transkriptoms vor Verabreichung der chemischen Verbindung eingegeben werden, das Maschinenlernmodell iterativ trainiert wird, um eine Differenz zwischen Daten des durch das Maschinenlernmodell ausgegebenen Ausmaßes der Transkriptomänderung und Daten einer Menge des Transkriptoms nach Verabreichung einer tatsächlichen chemischen Verbindung zu minimieren, und
wobei das Ausmaß der Transkriptomänderung die Differenz zwischen der Menge des Transkriptoms vor Verabreichung der chemischen Verbindung und der Menge des Transkriptoms nach Verabreichung der chemischen Verbindung darstellt.

**6.** Computer-implementiertes Verfahren nach Anspruch 5,
wobei das Maschinenlernmodell einen Abstand zwischen einem das Ausmaß der Transkriptomänderung darstellenden ersten Einbettungsvektor und einem Einbettungsvektor einer chemischen Verbindung, welche die Änderung der Menge des Transkriptoms induziert, durch eine Triplett-Verlustfunktion minimiert, und einen Abstand zwischen dem ersten Einbettungsvektor und dem Einbettungsvektor der chemischen Verbindung, welche die Änderung der Menge des Transkriptoms nicht induziert, maximiert.

**7.** Nicht-flüchtiges computerlesbares Medium, in welchem ein Programm zum Ausführen des Verfahrens nach einem der Ansprüche 1 bis 6 aufgezeichnet ist.

**Revendications**

**1.** Appareil destiné à délivrer un ou plusieurs nouveaux candidats-médicaments, comprenant :

un module de communication ;
une mémoire dans laquelle est stocké un programme destiné à fournir un ou plusieurs nouveaux candidats-médicaments ; et
un processeur exécutant le programme,
dans lequel le programme fournit un modèle d'apprentissage automatique dans lequel un vecteur d'embedding pour une structure chimique d'un composé chimique et un vecteur d'embedding pour une quantité d'un changement de transcriptome induit par chaque composé chimique sont situés dans un même espace vectoriel, délivre une quantité du changement de transcriptome qui correspond au vecteur d'embedding de la structure chimique du nouveau matériau entré dans le modèle d'apprentissage automatique, ou délivre un ou plusieurs médicaments qui correspondent au vecteur d'embedding pour la quantité du changement de transcriptome qui est entrée dans le modèle d'apprentissage automatique,

dans lequel, lorsque des données sur une quantité du transcriptome avant administration du composé chimique sont entrées, le modèle d'apprentissage automatique est entraîné de manière itérative pour minimiser une différence entre les données de la quantité du changement de transcriptome délivrées par le modèle d'apprentissage automatique et les données d'une quantité du transcriptome après administration d'un composé chimique réel, et

dans lequel la quantité du changement de transcriptome représente la différence entre la quantité du transcriptome avant administration du composé chimique et la quantité du transcriptome après administration du composé chimique.

2. Appareil selon la revendication 1,
dans lequel le modèle d'apprentissage automatique est configuré pour minimiser une distance entre un premier vecteur d'embedding représentant la quantité du changement de transcriptome et un vecteur d'embedding d'un composé chimique qui induit le changement dans la quantité du transcriptome au moyen d'une fonction de perte par triplet, et pour maximiser une distance entre le premier vecteur d'embedding et un vecteur d'embedding d'un composé chimique qui n'induit pas de changement dans la quantité du transcriptome.

3. Procédé mis en œuvre par ordinateur pour construire un modèle d'apprentissage automatique d'un dispositif informatique destiné à découvrir un ou plusieurs nouveaux candidats-médicaments, le procédé comprenant :

une étape consistant à construire un modèle d'apprentissage automatique dans lequel un vecteur d'embedding pour une structure chimique d'un composé chimique et un vecteur d'embedding pour une quantité d'un changement de transcriptome induit par chaque composé chimique sont situés dans un même espace vectoriel ; et

une étape consistant à exécuter un apprentissage itératif pour minimiser une différence entre des données sur une quantité du changement de transcriptome inférée par le modèle d'apprentissage automatique et des données sur une quantité du transcriptome après administration d'un composé chimique réel lorsque des données sur une quantité du transcriptome avant administration du composé chimique sont entrées dans le modèle d'apprentissage automatique, et

dans lequel la quantité du changement de transcriptome représente la différence entre la quantité du transcriptome avant administration du composé chimique et la quantité du transcriptome après administration du composé chimique.

4. Procédé mis en œuvre par ordinateur selon la revendication 3,
dans lequel, à l'étape de construction du modèle d'apprentissage automatique, la distance entre un premier vecteur d'embedding représentant la quantité du changement de transcriptome et un vecteur d'embedding d'un composé chimique qui induit ce changement est minimisée au moyen d'une fonction de perte par triplet, et une distance entre le premier vecteur d'embedding et un vecteur d'embedding d'un composé chimique qui n'induit pas de changement dans la quantité du transcriptome est maximisée.

5. Procédé mis en œuvre par ordinateur destiné à délivrer un ou plusieurs candidats-médicaments au moyen d'un processus d'inférence d'un modèle d'apprentissage automatique d'un dispositif informatique, le procédé comprenant :

une étape consistant à fournir un modèle d'apprentissage automatique dans lequel un vecteur d'embedding pour une structure chimique d'un composé chimique et un vecteur d'embedding pour une quantité d'un changement de transcriptome induit par chaque composé chimique sont situés dans un même espace vectoriel ;
une étape consistant à entrer un vecteur d'embedding pour une structure chimique d'un nouveau matériau ou un vecteur d'embedding pour une quantité d'un changement de transcriptome dans le modèle d'apprentissage automatique ; et
une étape consistant à délivrer, par le modèle d'apprentissage automatique, une quantité d'un changement de transcriptome qui correspond au vecteur d'embedding pour la structure chimique du nouveau matériau, ou à délivrer un ou plusieurs médicaments qui correspondent au vecteur d'embedding pour la quantité du changement de transcriptome,

dans lequel, lorsque des données sur une quantité du transcriptome avant administration du composé chimique sont entrées, le modèle d'apprentissage automatique est entraîné de manière itérative pour minimiser une différence entre les données de la quantité du changement de transcriptome délivrées par le modèle d'apprentissage automatique et les données d'une quantité du transcriptome après administration d'un composé chimique réel, et

dans lequel la quantité du changement de transcriptome représente la différence entre la quantité du transcriptome avant administration du composé chimique et la quantité du transcriptome après administration du composé chimique.

6. Procédé mis en œuvre par ordinateur selon la revendication 5,
dans lequel le modèle d'apprentissage automatique minimise une distance entre un premier vecteur d'embedding représentant la quantité du changement de transcriptome et un vecteur d'embedding d'un composé chimique qui induit le changement dans la quantité du transcriptome au moyen d'une fonction de perte par triplet, et maximise une distance entre le premier vecteur d'embedding et le vecteur d'embedding du composé chimique qui n'induit pas le changement dans la quantité du transcriptome.

7. Support non transitoire lisible par ordinateur sur lequel est enregistré un programme pour exécuter le procédé selon l'une quelconque des revendications 3 à 6.

# FIG. 1

*FIG. 2*

# FIG. 3

```
            ┌─────────────┐
            │    START    │
            └──────┬──────┘
                   │
                   ▼
    ┌──────────────────────────────┐
    │  CONSTRUCT DRUG LEARNING MODEL │──── S310
    └──────────────┬───────────────┘
                   │
                   ▼
    ┌──────────────────────────────┐
    │ INPUT CHEMICAL STRUCTURE OF    │
    │ NEW MATERIAL/CHANGE            │──── S320
    │ INFORMATION ON AMOUNT OF       │
    │ TRANSCRIPTOME TO DRUG          │
    │ LEARNING MODEL                 │
    └──────────────┬───────────────┘
                   │
                   ▼
    ┌──────────────────────────────┐
    │ OUTPUT RESULT OF CHANGE        │
    │ INFORMATION ON AMOUNT OF       │──── S330
    │ TRANSCRIPTOME/INFORMATION ON   │
    │ DRUG TO BE MATCHED             │
    └──────────────┬───────────────┘
                   │
                   ▼
            ┌─────────────┐
            │     END     │
            └─────────────┘
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020180058648
- US 20190114390 A1
- US 20190164632 A1

**Non-patent literature cited in the description**

- **YONI DONNER et al.** Drug repurposing using deep embeddings of gene expression profiles. *Molecular Pharmaceutics*, 2018, vol. 15 (10), 4314-4325
- **ALIPER ALEXANDER et al.** Deep Learning Applications for Predicting Pharmacological Properties of Drugs and Drug Repurposing Using Transcriptomic Data. *Molecular Pharmaceutics*, 2016, vol. 13 (7), 2524-2530